# EUROPÄISCHE PATENTSCHRIFT

(11) **EP 2 782 493 B1**
(45) Veröffentlichungstag und Bekanntmachung des Hinweises auf die Patenterteilung: **11.01.2023**
(21) Anmeldenummer: 12818491.8
(22) Anmeldetag: 22.11.2012
(51) Int. Cl.: A61B 3/032, A61H 5/00, A61B 3/113, A61B 5/00

(54) **VERFAHREN UND VORRICHTUNG ZUR VERBESSERUNG DER SEHLEISTUNG**
METHOD AND DEVICE IMPROVING VISUAL PERFORMANCE
PROCEDE ET DISPOSITIF PERMETTANT D'AMELIORER LES CAPACITÉS VISUELLES

(30) Priorität: 23.11.2011 DE 102011119361
(43) Veröffentlichungstag der Anmeldung: 01.10.2014
(73) Patentinhaber: Caterna Vision GmbH, 10117 Berlin (DE)
(72) Erfinder: LANGE, Robert, 01067 Dresden (DE); KÄMPF, Uwe, 01825 Liebstadt (DE); SEEWALD, Sascha, 01099 Dresden (DE)
(74) Vertreter: Harrison, Robert John
(86) Internationale Anmeldenummer: PCT/EP2012/073399
(87) Internationale Veröffentlichungsnummer: WO 2013/076211

(56) Entgegenhaltungen:
- WO-A2-2007/026368
- KR-A- 20010 103 223
- SU-A1- 982 655
- US-A1- 2005 099 601
- UWE KÄMPF ET AL: "Unterstützende Amblyopiebehandlung durch Computerspiele mit Hintergrundstimulation: Eine 10-tägige plazebokontrollierte Pilot-Studie = Amblyopia treatment by means of computer-games with background stimulation: a placebo controlled pilot study of 10 days", KLINISCHE MONATSBLAETTER FUER AUGENHEILKUNDE, FERDINAND ENKE VERLAG, STUTTGART, DE, Bd. 218, Nr. 4, 1. Januar 2001 (2001-01-01), Seiten 243-250, XP009167770, ISSN: 0023-2165, DOI: 10.1055/S-2001-14921
- U. POLAT ET AL: "Improving vision in adult amblyopia by perceptual learning", PROCEEDINGS OF THE NATIONAL ACADEMY OF SCIENCES, Bd. 101, Nr. 17, 1. April 2004 (2004-04-01), Seiten 6692-6697, XP055055713, ISSN: 0027-8424, DOI: 10.1073/pnas.0401200101
- SIMONS ET AL: "Amblyopia Characterization, Treatment, and Prophylaxis", SURVEY OF OPHTHALMOLOGY, SURVEY OF OPHTHALMOLOGY INC, XX, Bd. 50, Nr. 2, 1. März 2005 (2005-03-01), Seiten 123-166, XP027859519, ISSN: 0039-6257 [gefunden am 2005-03-01]

## Beschreibung

### Gebiet der Erfindung

Die vorliegende Erfindung betrifft eine Vorrichtung und ein System zur Verbesserung einer Sehleistung.

### Hintergrund der Erfindung

Der optokinetische Nystagmus (OKN) ist ein unwillkürlich entstehendes Bewegungsmuster der Augen, das z.B. durch, mit einer bestimmten Geschwindigkeit bewegte, optische Gitter mit einer bestimmten Ortsfrequenz und einer sinusoidalen Helligkeitsverteilung ausgelöst werden kann. Dieses Bewegungsmuster ist gekennzeichnet durch eine schnelle und eine langsame Phase. In der langsamen Phase folgen die Augen dem sich bewegenden Gitter und springen in der schnellen Phase nach einer bestimmten Elongation wieder schnell zurück, um dem Gitter darauf hin erneut zu folgen. Ein optokinetischer Nystagmus kann sowohl in horizontaler Richtung, als horizontaler optokinetischer Nystagmus (HOKN), als auch in vertikaler Richtung, als vertikaler optokinetischer Nystagmus (VOKN) auftreten, je nachdem ob das Bewegungsmuster des stimulierenden Gitters horizontal oder vertikal verläuft.

Wenn die dieses Bewegungsmuster auslösende Reizung unter der Bedingung geschieht, dass eines der Augen hinreichend bedeckt, d.h. okkludiert ist, entsteht bei dem nicht okkludierten Auge der monokulare optokinetische Nystagmus (MOKN).

Der OKN stellt ein charakteristisches Produkt bestimmter Leistungen des visuellen Systems einer Person dar. Seine Untersuchung kann daher relevante Informationen über eventuell vorhandene Beeinträchtigungen der Sehleistung, wie diese etwa bei Amblyopiepatienten auftreten, bieten. Weiterhin kann die gezielte Stimulation des OKN zur Verbesserung der einzelnen Sehleistungen führen, deren Zusammenwirken den OKN produzieren.

Als Amblyopie oder Schwachsichtigkeit wird die Sehschwäche eines (oder seltener beider) Augen bezeichnet, die auf einer unzureichenden Entwicklung des visuellen Systems während der frühen Kindheit beruht. Das Ergebnis ist eine Verminderung der Sehschärfe, die nicht oder zumindest nicht ausreichend durch organische Fehler erklärt werden kann, und die auch bei optimaler optischer Korrektur mit Brille oder Kontaktlinsen fortbesteht.

### Stand der Technik

Aus dem Stand der Technik sind bereits Systeme zur Messung und Verbesserung der Kontrastempfindlichkeit bzw. der Sehschärfe von Personen, bei denen diese Kontrastempfindlichkeit und die Sehschärfe nicht Normalwerten entsprechen, bekannt (US 8 002 409). Darin kann insbesondere für Amblyopen außerdem eine Klassifizierung der Amblyopie entsprechend der Hypothese über internes und externes Rauschen durchgeführt werden. Die Klassifizierung gemäß diesem Dokument basiert unter anderem auf der Arbeit von Andrew B. Watson "Detection and recognition of simple spatial forms" in "Physical and biological processing of images" herausgegeben von O. J. Braddick und A. C. Sleigh (Springer-Verlag, Berlin, 1983). Für die darin beschriebene Messung bzw. Training, sind vom Benutzer Eingaben über eine Benutzerschnittstelle zu tätigen. Zur Erzeugung der entsprechenden optischen Stimuli wird eine Hardware bereitgestellt, welche sich dazu eignet, aus einem standardisierten 3x8 Bit Farbvideosignal ein Graustufensignal mit höherer Auflösung als 8 Bit zu erzeugen.

Andere telemedizinische Systeme zur Messung und Verbesserung der Kontrastempfindlichkeit bzw. der Sehschärfe von Personen sind aus den Patenten von Neurovision bekannt. Unter anderem offenbaren die Patente US 7 004 912 und US 7 427 138 sowie die PCT Anmeldung WO 2007/043047 (Rabner) solche Systeme. Bei der PCT Anmeldung Nr. WO 2007/043047 handelt es sich um ein sogenanntes multifunktionales, optometrisch-ophthalmisches System zum Testen, zur Diagnose und zur Behandlung der Visionen oder des Auges einer Person. Das System umfasst eine Anordnung zum Anbringen auf einem Kopf und mindestens eine Augenmoduleinheit, welche an der Anordnung angebracht ist. Diese Moduleinheit erzeugt optische Reize, welche von zumindest einem Auge der Person erfasst sind. Die Augenmoduleinheit erhält die Ergebnisse der Reize und liefert sie an eine Zentralsteuerungs- und Verarbeitungseinheit. Die Augenmoduleinheit umfasst einen Mikroanzeiger, eine erste Linsenanordnung und eine Refraktionskorrekturanordnung. Das System kann für eine Vielzahl von optometrischen und opthalmischen Versuchen, für Diagnostika und für Behandlungen der Augen einer Person eingesetzt werden.

Aus Druckschrift KR 1020010103223 A ist ein System bekannt, das durch ein Programm für optokinetische Nystagmen eine OKN-Reaktion herleiten kann. Darin wird ein elektrookulografisches Verfahren zur Messung und Aufzeichnung der Augenbewegung verwendet. Dabei wird eine elektrische Spannung gemessen, was aber nur durch entsprechend geschulte Personen durchgeführt werden kann.

Weiterhin ist aus der Druckschrift GB 1 372 988 eine Vorrichtung bekannt, die zum Testen einer Sehschärfe unter Ausnutzung eines optokinetischen Nystagmus geeignet ist. Darin wird eine Kathodenstrahlröhre verwendet, deren Strahl so abgelenkt wird, dass ein vertikales Raster dargestellt wird. Es werden die Geschwindigkeit, der Kontrast und die Abmessungen des Rasters verändert. Neben der darin offenbarten Vorrichtung zur Erzeugung von einen optokinetischen Nystagmus auslösenden Stimuli wird auch die Detektion des Auftretens des optokinetischen Nystagmus durch Beobachtung oder durch ein Elektroenzephalogramm-Signal offenbart. Beide Varianten erfordern aber die zusätzliche Anwesenheit einer entsprechend geschulten Person.

Bei den aus dem Stand der Technik bekannten Systemen ist die Bedienung der Systeme durch eine entsprechend geschulte Person notwendig. Insbesondere sind hierbei nicht nur der Ablauf der Messung bzw. des Trainings von der Bedienung abhängig, sondern auch der Erfolg der Maßnahme kann maßgeblich von zu tätigenden Eingaben und von Mängeln derselben, d.h. fehlerhaften Eingaben, abhängen.

Aufgabe der vorliegenden Erfindung ist es, die Sehleistung einer Person zu verbessern, ohne auf die Bedienung durch eine entsprechend geschulte Person angewiesen zu sein.

### Kurzfassung der Erfindung

Die vorliegende Offenbarung offenbart eine Vorrichtung zur Verbesserung einer Sehleistung, mit einem ersten Anzeigeelement, zum Anzeigen von zumindest einem sich bewegenden, aus Gitterelementen bestehenden Gittern; zumindest einem tragbaren Verschlusselement, zur jeweiligen Okklusion eines Auges; einer tragbaren Blickerfassungseinrichtung, zur Messung zumindest eines charakteristischen Wertes eines nicht okkludierten Auges; einer ersten Steuereinheit, mit einer ersten Kommunikationsschnittstelle zu der Blickerfassungseinrichtung und einer zweiten Kommunikationsschnittstelle zu dem zumindest einem Verschlusselement; und einer zweiten Steuereinheit, die mit dem Anzeigeelement verbunden ist, mit einer dritten Kommunikationsschnittstelle zu der ersten Steuereinheit; wobei die zweite Steuereinheit dazu angepasst ist, eine erste Anzeige für ein nicht okkludiertes Auge der sich bewegenden Gitter durch das erste Anzeigeelement auf der Grundlage der durch die Blickerfassungseinrichtung gemessenen charakteristischen Werte zu steuern, und wobei die zweite Steuereinheit dazu angepasst ist, eine zweite Anzeige für ein nicht okkludiertes Auge auf dem ersten Anzeigeelement derart zu steuern, dass zusätzlich aufmerksamkeitsbindende Reize anzeigt werden.

Die vorliegende Offenbarung offenbart ein System zur Verbesserung einer Sehleistung, mit zumindest einer Vorrichtung, und einer Steuerungseinrichtung, mit einer dritten Steuereinheit, mit einem zweiten Anzeigeelement, zumindest einem Eingabegerät und zumindest einer vierten Kommunikationsschnittstelle zu den zweiten Steuereinheiten der zumindest einen Vorrichtung; wobei zumindest eine der zweiten Steuereinheiten weiterhin dazu angepasst ist, Betriebsdaten über den Betrieb der entsprechenden Vorrichtung zu sammeln und an die dritte Steuereinheit der Steuerungseinrichtung zu übertragen, Steuerdaten von der dritten Steuereinheit der Steuerungseinrichtung zu empfangen und den Betrieb der entsprechenden Vorrichtung unter Berücksichtigung der von der dritten Steuereinheit der Steuerungseinrichtung empfangenen Steuerdaten zu steuern, und wobei die dritte Steuereinheit dazu angepasst ist, die von der zumindest einen Vorrichtung übertragenen Betriebsdaten auf dem zweiten Anzeigeelement anzuzeigen und dazu angepasst ist, Steuerdaten, die auf einer über das zumindest eine Eingabegerät eingegebenen Eingabe basieren, an die zweite Steuerungseinheit der entsprechenden Vorrichtung zu senden.

Die Vorrichtung und das System werden für ein Verfahren zur Verbesserung einer Sehleistung verwendet werden. Das Verfahren umfasst Okkludieren eines Auges durch ein Verschlusselement, Messen zumindest eines charakteristischen Wertes eines nicht okkludierten Auges durch eine Blickerfassungseinrichtung, und Anzeigen für ein nicht okkludiertes Auge von sich bewegenden Gittern und von aufmerksamkeitsbindenden Reizen auf einem Anzeigeelement, der durch eine Steuereinheit auf der Grundlage der durch die Blickerfassungseinrichtung gemessenen charakteristischen Werte gesteuert wird.

Durch die vorliegende Erfindung, die durch die unabhängigen Ansprüche definiert wird, können Messungen automatisiert ausgelöst werden und auch automatisierte Datenanalysen durchgeführt werden. Die gewonnen Messdaten können auch direkt nutzbar gemacht werden, wodurch die Effizienz der Verbesserung der Sehleistung gesteigert werden kann. Durch die automatische Messung können weiterhin Daten gemessen werden, die längerfristige Veränderungen des Zustandes der Sehleistung nachvollziehbar machen.

### Kurzbeschreibung der Zeichnungen

Es zeigen:
Fig. 1 eine Vorrichtung zur Verbesserung der Sehleistung,
Fig. 2 ein System zur Verbesserung der Sehleistung, und
Fig. 3 ein Flussdiagramm eines Verfahrens zur Verbesserung der Sehleistung.

### Ausführliche Beschreibung

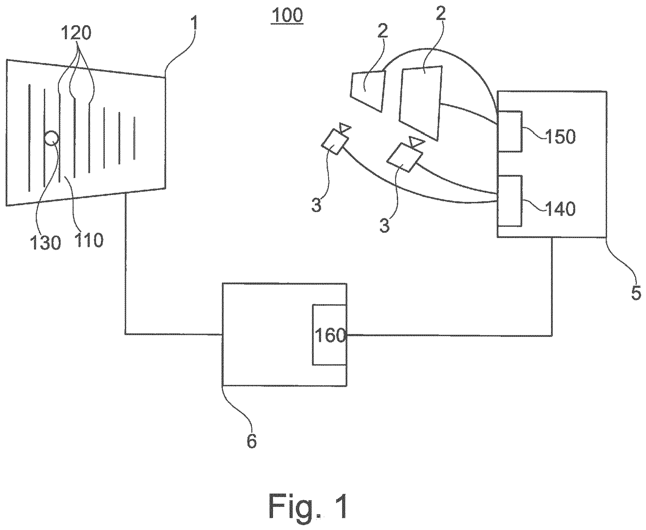

Fig. 1 zeigt eine Vorrichtung 100 zur Verbesserung der Sehleistung eines Betrachters. Die Vorrichtung 100 umfasst ein erstes Anzeigeelement 1. Auf diesem Anzeigeelement I werden Gitter 110, die aus Gitterelementen 120 bestehen, und sogenannte aufmerksamkeitsbindende Reize 130, d.h. optisch-visuelle Reize, welche die Aufmerksamkeit eines Betrachters binden, angezeigt. Diese aufmerksamkeitsbindenden Reize können verschiedener Art sein, jegliche geometrische Form aufweisen und entweder in Farbe oder monochrom dargestellt werden. Es können aber auch andere Reize, die geeignet sind die Aufmerksamkeit des Betrachters zu binden, verwendet werden. Um die Augen eines Betrachters unabhängig voneinander den sich bewegenden Gittern und den aufmerksamkeitsbindenden Reizen aussetzen zu können, gibt es tragbare Verschlusselemente, mit denen jeweils ein Auge des Betrachters abgedeckt, d.h. okkludiert werden kann. Für das jeweils nicht okkludierte Auge steht eine tragbare Blickerfassungseinrichtung 3 zur Verfügung, mit der charakteristische Werte des nicht okkludierten Auges gemessen werden können.

Das Zusammenspiel der Blickerfassungseinrichtungen 3 und der tragbaren Verschlusselemente 2 wird durch eine erste Steuereinheit 5 gesteuert. Diese erste Steuereinheit 5 weist dazu eine erste Kommunikationsschnittstelle 140 zu den Blickerfassungseinrichtungen 3 und eine zweite Kommunikationsschnittstelle 150 zu den Verschlusselementen 2 auf. Eine Anzeige auf dem ersten Anzeigeelement 1 wird durch eine zweite Steuereinheit 6, die mit dem ersten Anzeigeelement 1 verbunden ist, gesteuert. Die zweite Steuereinheit 6 hat eine dritte Kommunikationsschnittstelle 160 zur ersten Steuereinheit 5 und steuert die Anzeige auf dem Anzeigeelement 1 auf der Grundlage der charakteristischen Werte, die durch die tragbaren Blickerfassungseinrichtungen 3 erfasst worden sind.

Die Verschlusselemente 2 können z.B. elektronisch steuerbare Flüssigkristall-Verschlüsse sein, die auch LC-Shutter genannt werden. Die Blickerfassungseinrichtungen 3 können z.B. tragbare Videookulographie-Geräte, die auch VOG-Geräte genannt werden, Infrarotokulographie-Geräte, die auch IROG-Geräte genannt werden oder eine Kombination aus beiden sein..

Um die Benutzung der Vorrichtung 100 für den Betrachter einfach und angenehm zu gestalten, können die erste Steuereinheit 5, die Blickerfassungseinrichtungen 3 und die Verschlusselemente 2 in einer Benutzereinheit zusammengefasst werden, die zudem nach Art einer Brille aufgesetzt werden kann. Um die Benutzung der Vorrichtung 100 auch Personen zu ermöglichen, die weitere Augenleiden haben, kann diese Benutzereinheit zudem Einschübe aufweisen, die zur Aufnahme von Refraktionslinsen geeignet sind, um damit die Vorrichtung 100 auf die Augenwerte des Betrachters anpassen zu können.

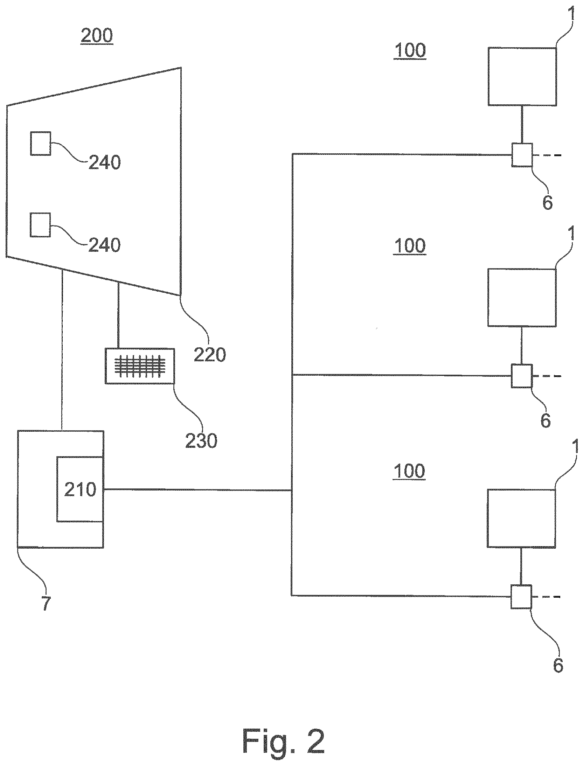

Fig. 2 zeigt ein System, welches die Vorrichtungen 100 zeigt, die in Fig. 1 dargestellt sind. Darüber hinaus weist das System eine Steuerungseinrichtung 200 auf, die über eine vierte Kommunikationsschnittstelle 210 mit den jeweiligen zweiten Steuereinheiten 6 der Vorrichtungen 100 verbunden ist und über diese Verbindung mit den Vorrichtungen 100 kommunizieren kann. Außerdem sind für die Bedienung der Steuerungseinrichtung 200 ein zweites Anzeigeelement 220 und ein Eingabegerät 230 angeschlossen. Über die Kommunikationsverbindung werden Betriebsdaten 240 der Vorrichtungen 100 an die Steuerungseinrichtung 200 übermittelt. Die Betriebsdaten 240 wurden zuvor von den jeweiligen zweiten Steuereinheiten 6 der Vorrichtungen 100 gesammelt.

An der Steuerungseinrichtung 200 werden die Betriebsdaten 240 auf dem zweiten Anzeigeelement 220 dargestellt, und über das Eingabegerät 230 können Eingaben getätigt werden, die der Steuerung der Vorrichtungen 100 dienen. In Gegenrichtung zu der vorherigen Datenübermittlung der Betriebsdaten 240 werden von der Steuerungseinrichtung 200 Steuerdaten an die Vorrichtungen 100 gesendet, dort von den zweiten Steuereinheiten 6 empfangen und beim Betrieb der jeweils eigenen Vorrichtung 100 berücksichtigt.

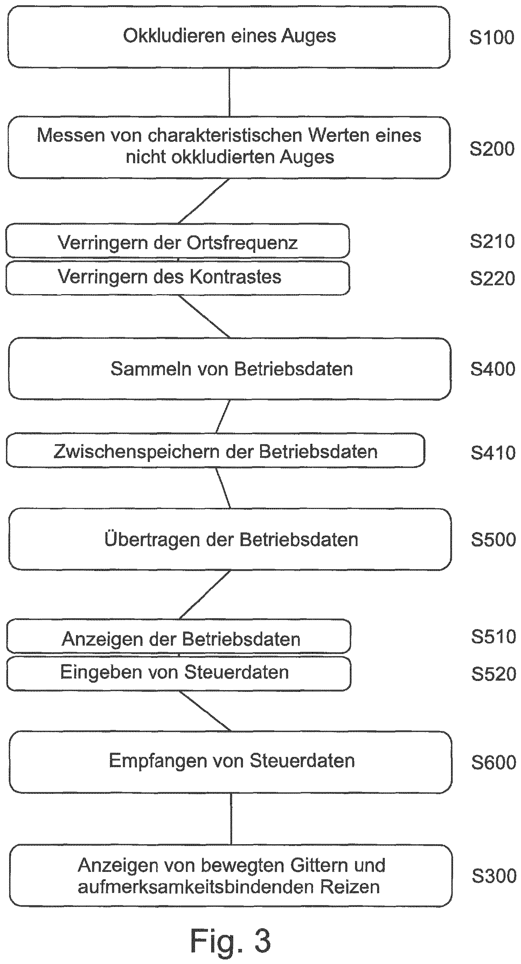

Fig. 3 zeigt ein Flussdiagramm eines Verfahrens zur Verbesserung der Sehleistung. Dabei wird in Schritt S100 ein Auge des Betrachters durch das tragbare Verschlusselement 2 okkludiert, d.h. lichtundurchlässig abgedeckt. Im Anschluss daran wird im Schritt S200 zumindest ein charakteristischer Wert des nicht abgedeckten Auges des Betrachters durch die Blickerfassungseinrichtung 3 gemessen. Schließlich werden in Schritt S300 für ein nicht okkludiertes Auge auf dem ersten Anzeigeelement 1 die sich bewegenden Gitter 110 und die aufmerksamkeitsbindenden Reize 130 angezeigt. Diese Anzeige auf dem ersten Anzeigeelement 1 wird durch die Steuereinheit 6 auf der Grundlage der durch die Blickerfassungseinrichtung 3 gemessenen charakteristischen Werte gesteuert. Dabei werden unter Berücksichtigung der gemessenen charakteristischen Werte unter anderem die Ortsfrequenz und der Kontrast der Anzeige der Gitter gesteuert.

Beim Messen in Schritt S200 können dabei beispielsweise zwei charakteristische Werte gemessen werden, indem im Schritt S210 zuerst ein Verringern der Ortsfrequenz der sich bewegenden Gitter 110 auf dem ersten Anzeigeelement 1 durchgeführt wird, bis ein monokularer optokinetischer Nystagmus (MOKN) auftritt. Der dabei eingestellte Wert der Ortsfrequenz wird dabei als erster charakteristischer Wert gespeichert.

Daraufhin wird in Schritt S220 der Kontrast der Anzeige der sich bewegenden Gitter 110 auf dem ersten Anzeigeelement 1 verringert, bis der monokulare optokinetische Nystagmus (MOKN) aussetzt. Der dabei eingestellte Wert des Kontrastes wird dabei als zweiter charakteristischer Wert gespeichert.

Ein Messen gemäß Schritt S200 wird für jedes Auge durchgeführt. Das Messen kann für jedes Auge mit Gittern wiederholt werden deren Bewegungsablauf um 90°, 180° und 270° gedreht ist. Dabei wird jedes Mal der Bewegungsverlauf des jeweiligen optokinetischen Nystagmus aufgezeichnet, solange dieser auftritt.

Wird das oben beschriebene System eingesetzt, so werden in Schritt S400 Betriebsdaten 240 über den Ablauf des Verfahrens auf der Vorrichtung 100 von der Steuereinheit 6 der Vorrichtung 100 gesammelt und in Schritt S500 an die Steuerungseinrichtung 200 übertragen. Unter Umständen werden diese Betriebsdaten 240 in Schritt S410 zwischengespeichert.

Auf dem zweiten Anzeigeelement 220 der Steuerungseinrichtung 200 werden in Schritt S510 die von den Vorrichtungen 100 übertragenen Betriebsdaten 240 angezeigt, und es wird einem Benutzer der Steuerungseinrichtung 200 in Schritt S520 ermöglicht, durch eine Eingabe auf den Ablauf des Verfahrens in den Vorrichtungen 100 Einfluss zu nehmen. Die hierbei eingegebenen Daten, z.B. Steuerungsparameter oder Steuerdaten 250, werden in Schritt S600 wiederum an die Vorrichtungen 100 gesendet, wo die empfangenen Steuerdaten 250 dann in Schritt S300 bei der Anzeige der sich bewegenden Gitter 110 berücksichtigt werden.

Durch die Vorrichtung 100 gemäß Fig. 1 kann ein Fixationsvermögen von Personen (Betrachtern), darunter auch Amblyopen, bei denen das Fixationsvermögen gestört ist, trainiert werden. Die Vorrichtung 100 ermöglicht eine Verbesserung des okulomotorischen Verhaltens sowie (damit verbunden) einen Anstieg des Visus. Da bei Amblyopen die monokularen optokinetischen Nystagmen (OKN) üblicherweise asymmetrisch sind, kann der Grad der Asymmetrie als indirektes Messverfahren zur Beurteilung des Fixationsvermögens sowie des Vermögens zur beidäugigen Zusammenarbeit verwendet werden. Der Asymmetrie-Grad und sein Verlauf während der Benutzung der Vorrichtung kann algorithmengestützt aus den Augenbewegungsdaten ermittelt werden und über das zweite Anzeigenelement 220 der Steuerungseinrichtung 200 angezeigt werden. Versuchsreihen haben ergeben, dass eine signifikante Verbesserung der Sehleistung nach einer Benutzung über 3 Monate hinweg eintreten kann, wenn die Vorrichtung täglich für etwa 20 Minuten verwendet wird.

Die Stimulation mit den sich bewegenden Gittern 110, welche den optokinetischen Nystagmus auslösen, führt zur Verbesserung des Fixationsvermögens und damit auch zur Erhöhung des Visus. Diese Verbesserung stellt sich auch ein, wenn das Auftreten des optokinetischen Nystagmus durch Fixation unterdrückt wird, also z.B. durch Fixieren von aufmerksamkeitsbindenden Reizen.

Zur (indirekten) Messung wird keine Eingabe vom Betrachter erwartet, sondern es wird ein unwillkürliches Bewegungsmuster, nämlich der monokulare optokinetische Nystagmus der Augen, welcher durch einen entsprechenden optischen Stimulus in Form von sich bewegenden Gittern ausgelöst wird, mit Hilfe der Blickerfassungseinrichtung 3 sowie des tragbaren Verschlusselementes 2 gemessen.

Bei Betrachtern mit einer Sehschwäche kann es vorkommen, dass das Auslösen des OKN je nach Art und Umfang der Sehschwäche im Vergleich zum Normalsichtigen nur bei sich bewegenden Gittern 110 mit hinreichend niedriger Ortsfrequenz und hinreichend starkem Kontrast eintritt. Bei Betrachtern mit Amblyopie (Sehschwäche eines Auges oder seltener beider Augen, die auf einer unzureichenden Entwicklung des Sehsystems der Person während der frühen Kindheit beruht) gilt dies im Allgemeinen und hängt von der Stärke der Amblyopie ab. Personen die an Amblyopie leiden werden auch Amblyopen genannt.

Gegenüber einem Normalsichtigen können ausgeprägte Asymmetrien zwischen den MOKN vorkommen. Asymmetrisch kann z.B. bedeuten, dass sich die langsamen Phasen in Abhängigkeit von der Bewegungsrichtung des sich bewegenden Gitters 110 unterscheiden. Für die Bewegung gibt es jeweils zwei Richtungen: von der Schläfe zur Nase (von temporal nach nasal) und umgekehrt, von der Nase zur Schläfe (von nasal nach temporal). Ein Unterschied liegt dabei darin, dass sich die Winkelgeschwindigkeit des Auges in der langsamen Phase unterscheidet.

Diese Asymmetrien existieren auch beim Normalsichtigen, jedoch verschwinden diese zumeist sobald eine sichere binokulare Zusammenarbeit im Verlauf der postnatalen Ontogenese des visuellen Systems erreicht wurde. Beim Amblyopen allerdings bleibt diese Asymmetrie besonders auf dem sog. amblyopen Auge bestehen, und resultiert darin, dass sich die zwei MOKN einer Person unterscheiden.

Ein Gitterstimulus, welcher einen optokinetischen Nystagmus auslösen kann, kann durch einen weiteren Reiz im Vordergrund ergänzt werden, der die Aufmerksamkeit des Betrachters auf sich ziehen kann. Dabei kommt es zu einem passagerem (vorübergehenden) Fixationswandel und dadurch kann bei wiederholter Darbietung in Form eines Trainings die Ausbildung eines stabilen Fixationswandels unterstützt werden, welcher bei Amblyopen oft gestört ist.

So ein Reiz, der die Aufmerksamkeit auf sich ziehen kann, kann z.B. in Form von geeigneten Spielen dargeboten werden. Für den Vordergrundreiz gilt die Voraussetzung, dass dieser den Gitterstimulus nicht dauerhaft großflächig abdeckt (im Mittel maximal bis zu 10%).

Zusammenfassend lässt sich feststellen, dass der OKN einerseits dazu dienen kann, die Stärke und Form einer Amblyopie in Bezug auf eine okulomotorische Leistung zu beurteilen und andererseits kann er in Verbindung mit aufmerksamkeitsbindenden Vordergrundreizen zu einem, die Stärke der Amblyopie nachhaltig mindernden Trainingseffekt führen.

**Bezugszeichen**

| | |
|---|---|
| 1 | Erstes Anzeigeelement |
| 2 | Tragbare Verschlusselemente |
| 3 | Blickerfassungeinrichtung |
| 5 | Erste Steuereinheit |
| 6 | Zweite Steuereinheit |
| 7 | Dritte Steuereinheit |
| | |
| 100 | Vorrichtung |
| 110 | Sich bewegende Gitter |
| 120 | Gitterelemente |
| 130 | Aufmerksamkeitsbindende Reize |
| 140 | Erste Kommunikationsschnittstelle |
| 150 | Zweite Kommunikationsschnittstelle |
| 160 | Dritte Kommunikationsschnittstelle |
| | |
| 200 | Steuerungseinrichtung |
| 210 | Vierte Kommunikationsschnittstelle |
| 220 | Zweites Anzeigeelement |
| 230 | Eingabegerät |
| 240 | Betriebsdaten |
| 250 | Steuerdaten |

## Patentansprüche

1. Vorrichtung (100) zur Verbesserung einer Sehleistung, mit
einem ersten Anzeigeelement (1), dazu angepasst, zumindest ein sich bewegendes, aus Gitterelementen (120) bestehendes Gitter (110) anzuzeigen;
zumindest einem tragbaren Verschlusselement (2), zur Okklusion mindestens eines Auges;
einer tragbaren Blickerfassungseinrichtung (3), zur Messung zumindest eines charakteristischen Wertes eines nicht okkludierten Auges;
einer ersten Steuereinheit (5), mit einer ersten Kommunikationsschnittstelle (140) zu der Blickerfassungseinrichtung (3) und einer zweiten Kommunikationsschnittstelle (150) zu dem zumindest einem Verschlusselement (2); und
einer zweiten Steuereinheit (6), die mit dem Anzeigeelement (1) verbunden ist, mit einer dritten Kommunikationsschnittstelle (160) zu der ersten Steuereinheit (5);
wobei die zweite Steuereinheit (6) dazu angepasst ist, eine Anzeige für ein nicht okkludiertes Auge der sich bewegenden Gitter (110) durch das erste Anzeigeelement (1) auf der Grundlage der durch die Blickerfassungseinrichtung (3) gemessenen charakteristischen Werte zu steuern, und
wobei die zweite Steuereinheit (6) dazu angepasst ist, eine Anzeige für ein nicht okkludiertes Auge auf dem ersten Anzeigeelement (1) derart zu steuern, dass zusätzlich aufmerksamkeitsbindende Reize (130) anzeigt werden.

2. Vorrichtung (100) gemäß Anspruch 1, wobei die Blickerfassungseinrichtung (3) dazu eingerichtet ist, eine Ortsfrequenz und einen Kontrast des sich bewegenden Gitters, als die charakteristischen Werte derart zu messen, dass zuerst die Ortsfrequenz des sich bewegenden Gitters (110) auf dem ersten Anzeigeelement (1) verringert wird bis ein monokularer optokinetischer Nystagmus (MOKN) auftritt, und dann der Kontrast der ersten Anzeige des sich bewegenden Gitter (110) verringert wird, bis der monokulare optokinetische Nystagmus (MOKN) aussetzt.

3. Vorrichtung (100) gemäß einem der vorstehenden Ansprüche, wobei das zumindest eine tragbare Verschlusselement (2) ein elektronisch steuerbarer Flüssigkristall-Verschluss (LC-Shutter) ist.

4. Vorrichtung (100) gemäß einem der vorstehenden Ansprüche, wobei die Blickerfassungseinrichtung (3) ein tragbares Videookulographie-Gerät (VOG-Gerät), ein tragbares Infrarotokulographie-Gerät (IROG-Gerät), oder eine Kombination aus Videookulographie-Gerät und Infrarotokulographie-Gerät ist.

5. Vorrichtung (100) gemäß einem der vorstehenden Ansprüche, wobei die erste Steuereinheit (5), die Blickerfassungseinrichtung (3) und das zumindest eine Verschlusselement (2) in einer Benutzereinheit zusammengefasst sind.

6. Vorrichtung (100) gemäß Anspruch 5, wobei die Benutzereinheit wie eine Brille aufgesetzt werden kann.

7. Vorrichtung (100) gemäß Anspruch 5 oder 6, wobei die Benutzereinheit Einschübe zur Aufnahme von Refraktionslinsen aufweist.

8. System zur Verbesserung einer Sehleistung, mit
zumindest einer Vorrichtung (100) gemäß einem der Ansprüche 1 bis 7, und
einer Steuerungseinrichtung (200), mit einer dritten Steuereinheit (7), mit einem zweiten Anzeigeelement (220), zumindest einem Eingabegerät (230) und zumindest einer vierten Kommunikationsschnittstelle (210) zu den zweiten Steuereinheiten (6) der zumindest einen Vorrichtung (100);
wobei zumindest eine der zweiten Steuereinheiten (6) weiterhin dazu angepasst ist, Betriebsdaten (240) über den Betrieb der entsprechenden Vorrichtung (100) zu sammeln und an die dritte Steuereinheit (7) der Steuerungseinrichtung (200) zu übertragen, Steuerdaten (250) von der dritten Steuereinheit (7) der Steuerungseinrichtung (200) zu empfangen und den Betrieb der entsprechenden Vorrichtung (100) unter Berücksichtigung der von der dritten Steuereinheit (7) der Steuerungseinrichtung (200) empfangenen Steuerdaten (250) zu steuern, und
wobei die dritte Steuereinheit (7) dazu angepasst ist, die von der zumindest einen Vorrichtung (100) übertragenen Betriebsdaten (240) auf dem zweiten Anzeigeelement (220) anzuzeigen und dazu angepasst ist, Steuerdaten (250), die auf einer über das zumindest eine Eingabegerät (230) eingegebenen Eingabe basieren, an die zweite Steuerungseinheit (6) der entsprechenden Vorrichtung (100) zu senden.

9. System gemäß Anspruch 8, wobei die von einer der zweiten Steuereinheiten (6) gesammelten Betriebsdaten (240) in der jeweiligen zweiten Steuereinheit (6) zwischengespeichert werden.

10. Verwendung einer Vorrichtung (100) oder eines Systems gemäß einem der Ansprüche 1 bis 9 zur Verbesserung einer Sehleistung.

## Claims

1. Device (100) for improving a visual performance, having
a first display element (1), adapted for displaying at least one moving grid (110) consisting of grid elements (120)
at least one portable closure element (2), for occluding at least one eye;
a portable eye tracking device (3), for measuring at least one characteristic value of a non-occluded eye;
a first control unit (5) having a first communication interface (140) to the eye tracking device (3) and a second communication interface (150) to the at least one closure element (2); and
a second control unit (6) connected to the display element (1), having a third communication interface (160) to the first control unit (5);
wherein the second control unit (6) is adapted to control a display for a non-occluded eye of the moving grids (110) by the first display element (1) based on the characteristic values measured by the eye tracking device (3), and
wherein the second control unit (6) is adapted to control a display for a non-occluded eye on the first display element (1) in such a way that attention-capturing stimuli (130) are additionally displayed.

2. Device (100) according to claim 1, wherein the eye tracking device (3) is adapted to measure a spatial frequency and a contrast of the moving grid, as the characteristic values, such that first the spatial frequency of the moving grid (110) on the first display element (1) is decreased until a monocular optokinetic nystagmus (MOKN) occurs, and then the contrast of the first display of the moving grid (110) is decreased until the monocular optokinetic nystagmus (MOKN) ceases.

3. Device (100) according to any one of the preceding claims, wherein the at least one portable closure element (2) is an electronically controllable liquid crystal shutter (LC shutter).

4. Device (100) according to any one of the preceding claims, wherein the eye tracking device (3) is a portable video-oculography device (VOG device), a portable infrared-oculography device (IROG device), or a combination of video-oculography device and infrared-oculography device.

5. Device (100) according to any one of the preceding claims, wherein the first control unit (5), the eye tracking device (3), and the at least one closure element (2) are combined in one user unit.

6. Device (100) according to claim 5, wherein the user unit can be put on like a pair of glasses.

7. Device (100) according to claim 5 or 6, wherein the user unit comprises inserts for receiving refractive lenses.

8. System for improving a visual performance, having
at least one device (100) according to any one of claims 1 to 7, and
a control device (200), having a third control unit (7), having a second display element (220), at least one input device (230), and at least one fourth communication interface (210) to the second control units (6) of the at least one device (100);
wherein at least one of the second control units (6) is further adapted to collect and transmit operating data (240) about the operation of the corresponding device (100) to the third control unit (7) of the control device (200), to receive control data (250) from the third control unit (7) of the control device (200), and to control the operation of the corresponding device (100) taking into account the control data (250) received from the third control unit (7) of the control device (200), and
wherein the third control unit (7) is adapted for displaying the operating data (240) transmitted from the at least one device (100) on the second display element (220) and is adapted to send control data (250) based on an input entered via the at least one input device (230) to the second control unit (6) of the corresponding device (100).

9. System according to claim 8, wherein the operating data (240) collected by one of the second control units (6) is temporarily stored in the respective second control unit (6).

10. Use of a device (100) or system according to any one of claims 1 to 9 for improving a visual performance.

## Revendications

1. Dispositif (100) destiné à améliorer une performance visuelle, comprenant
un premier élément d'affichage (1) adapté pour afficher au moins une grille mobile (110) composée d'éléments de grille (120) ;
au moins un élément d'occlusion portable (2), pour l'occlusion d'au moins un oeil ;
un dispositif de détection de regard (3) portable, pour mesurer au moins une valeur caractéristique d'un œil non occlus ;
une première unité de commande (5), avec une première interface de communication (140) avec le dispositif de détection du regard (3) et une deuxième interface de communication (150) avec le au moins un élément d'occlusion (2) ; et
une deuxième unité de commande (6), qui est reliée à l'élément d'affichage (1), avec une troisième interface de communication (160) avec la première unité de commande (5) ;
dans lequel la deuxième unité de commande (6) est adaptée pour commander un affichage, pour un œil non occlus, des grilles mobiles (110) par le premier élément d'affichage (1) sur la base des valeurs caractéristiques mesurées par le dispositif de détection de regard (3), et
dans lequel la deuxième unité de commande (6) est adaptée pour commander un affichage, pour un œil non occlus, sur le premier élément d'affichage (1) de telle sorte que des stimuli (130) attirant l'attention soient affichés en plus.

2. Dispositif (100) selon la revendication 1, dans lequel le moyen de détection de regard (3) est adapté pour mesurer une fréquence spatiale et un contraste de la grille mobile, en tant que valeurs caractéristiques, de sorte que d'abord la fréquence spatiale de la grille mobile (110) sur le premier élément d'affichage (1) est réduite jusqu'à ce qu'un nystagmus optocinétique monoculaire (MOKN) se produise, et ensuite le contraste du premier affichage de la grille mobile (110) est réduit jusqu'à ce que le nystagmus optocinétique monoculaire (MOKN) s'arrête.

3. Dispositif (100) selon l'une quelconque des revendications précédentes, dans lequel ledit au moins un élément d'obturation portable (2) est un obturateur à cristaux liquides (LC-Shutter) à commande électronique.

4. Dispositif (100) selon l'une quelconque des revendications précédentes, dans lequel le dispositif de détection de regard (3) est un appareil de vidéo-oculographie portable (appareil VOG), un appareil d'oculographie infrarouge portable (appareil IROG), ou une combinaison d'un appareil de vidéo-oculographie et d'un appareil d'oculographie infrarouge.

5. Dispositif (100) selon l'une des revendications précédentes, dans lequel la première unité de commande (5), le dispositif de détection du regard (3) et le au moins un élément d'obturation (2) sont regroupés dans une unité utilisateur.

6. Dispositif (100) selon la revendication 5, dans lequel l'unité utilisateur peut être mise en place comme une paire de lunettes.

7. Dispositif (100) selon la revendication 5 ou 6, dans lequel l'unité utilisateur comprend des inserts pour recevoir des lentilles de réfraction.

8. Système d'amélioration d'une performance visuelle, comprenant
au moins un dispositif (100) selon l'une des revendications 1 à 7, et
un dispositif de commande (200), avec une troisième unité de commande (7), avec un deuxième élément d'affichage (220), au moins un appareil d'entrée (230) et au moins une quatrième interface de communication (210) avec les deuxièmes unités de commande (6) du au moins un dispositif (100) ;
dans lequel au moins l'une des deuxièmes unités de commande (6) est en outre adaptée pour collecter et transmettre à la troisième unité de commande (7) du dispositif de commande (200) des données de fonctionnement (240) concernant le fonctionnement du dispositif correspondant (100), recevoir des données de commande (250) de la troisième unité de commande (7) du dispositif de commande (200), et commander le fonctionnement du dispositif correspondant (100) en tenant compte des données de commande (250) reçues de la troisième unité de commande (7) du dispositif de commande (200), et
dans lequel la troisième unité de commande (7) est adaptée pour afficher sur le deuxième élément d'affichage (220) les données de fonctionnement (240) transmises par le au moins un dispositif (100) et est adaptée pour envoyer à la deuxième unité de commande (6) du dispositif correspondant (100) des données de commande (250) basées sur une entrée introduite via le au moins un dispositif d'entrée (230).

9. Système selon la revendication 8, dans lequel les données de fonctionnement (240) collectées par l'une des deuxièmes unités de commande (6) sont stockées temporairement dans la deuxième unité de commande (6) respective.

10. Utilisation d'un dispositif (100) ou d'un système selon l'une quelconque des revendications 1 à 9 pour améliorer une performance visuelle.
